# EUROPEAN PATENT APPLICATION

(11) **EP 4 335 371 A1**
(43) Date of publication of application: **13.03.2024**
(21) Application number: 23195753.1
(22) Date of filing: 06.09.2023
(51) Int. Cl.: A61B 5/257, A61B 5/265, A61B 5/27, A61B 5/00, A61N 1/04, A61N 1/18

(54) **MALLEABLE ELECTRODE**

(30) Priority: 06.09.2022 LU 502755
(71) Applicant: InnovationLab GmbH, 69115 Heidelberg (DE)
(72) Inventor: Kaiser, Christoph, 69115 Heidelberg (DE)
(74) Representative: Harrison, Robert John

(57) **Abstract**

A malleable electrode comprises a malleable substrate and a plurality of conducting paths. The plurality of conducting paths is disposed with a non-linear shape on a surface of the substrate. At least two adjacent ones of the plurality of conducting paths are lengthwise offset from each other and the at least two adjacent ones of the plurality of conductive paths are in contact with each other at oppositely disposed contact locations.

## Description

### FIELD OF THE INVENTION

This application claims priority of Luxembourg Patent Application number LU502755, filed on 6 September 2022. The entire disclosure of the Luxembourg Patent Application number LU502755 is hereby incorporated herein by reference.

The field of the invention relates to a malleable electrode, an item of clothing comprising the malleable electrode, and a method for manufacturing the malleable electrode.

### BACKGROUND OF THE INVENTION

Electrodes are used in a variety of application areas and have a number of different electrode designs. The field of textile integrated electronics is a rapidly growing application field. The electrodes are integrated into the textiles and can interact with a human body. The stimulation of muscles is just one possible application of the textile integrated electronics.

In the publication *"*Wearable Electrical Stimulation to Improve Lymphatic Function" (Y. Wei, K. Yang, M. Browne, L. Bostan and P. Worsley, in IEEE Sensors Letters, vol. 3, no. 2, pp. 1-4, Feb. 2019, Art no. 2500604, doi: 10.1109/LSENS.2019.2893478.), textile electrodes produced by screen printing are described. Individual planar electrodes, with a lead, are printed on a textile substrate, from silver-containing ink. The individual planar electrodes are coated with a flat coating as a contact surface to the skin. The coating is produced by screen printing using carbon-containing ink.

The prior art discloses electrodes with a planar design. The planar design leads to the electrodes having a significant degree of stiffness, as the electrodes comprise a solid piece of material. The stiffness of the electrodes brings certain disadvantages when integrating the electrodes into textiles. For example, wearing items of clothing with the integrated stiff electrodes can be uncomfortable for the wearer. The stiffness can also prevent a close fit between the electrode and the skin of the wearer, especially when the wearer is moving. Other ones of the prior art electrodes are bendable. However, the prior art does not disclose electrodes that are malleable, i.e., both stretchable in at least one direction and bendable.

### SUMMARY OF THE INVENTION

This document discloses malleable electrodes that can be bent, twisted or stretched without risking damage to the electrode. An item of clothing which includes the malleable electrode and a method for manufacturing the malleable electrode are further described.

According to a first aspect of the invention, the malleable electrode comprises a malleable substrate and a plurality of conducting paths. The plurality of conducting paths are disposed on a surface of the substrate with a non-linear shape in a plane of the surface and at least two adjacent ones of the plurality of conducting paths are lengthwise offset from each other. The adjacent ones of the plurality of conductive paths are in contact with each other at oppositely disposed contact locations.

The plurality of conducting paths can further have a wave-shaped form with a plurality of apexes. The adjacent ones of the plurality of conducting paths can contact each other at respective opposing ones of the plurality of apexes.

According to another aspect of the invention, the plurality of conducting paths form a grid structure.

The conducting paths can be made of silver-containing material and can further be at least partially coated with a coating made of a carbon-containing material.

According to another aspect of the invention, the plurality of conducting paths are printed on the substrate using silver-containing ink. The coating of the plurality of conducting paths may further be printed using carbon-containing ink.

The substrate of the malleable electrode can comprise a first layer made of thermoplastic polyurethane. The substrate of the malleable electrode further comprises a second layer made of hotmelt adhesives. The substrate can further be perforated.

The plurality of conducting paths are further connectable at a plurality of locations to electrical devices, such as but not limited to a power source or a measurement device.

In another aspect of the invention, an item of clothing including the malleable electrode is described. At least one patch of the malleable electrode is disposed on an area of the clothing. At least one of the plurality of conducting paths is further contactable with a skin of a wearer of the clothing. Alternatively, the coating of at least one of the plurality of conducting paths is contactable with a skin of a wearer of the clothing.

The items of clothing further comprises a power source electrically connected to at least one of the plurality of conducting paths of at least one patch of the malleable electrode. The clothing further comprises a current measurement device electrically connected to at least one of the plurality of conducting paths of at least one patch of the malleable electrode.

A method of manufacturing a malleable electrode is further described. The method comprises the steps of providing a malleable substrate and creating a plurality of conducting paths on a surface of the malleable substrate. The created plurality of conducting paths are thereby arranged with a non-linear shape in a plane of the surface and at least two adjacent ones of the plurality of conducting paths are lengthwise offset from each other. The at least two adjacent ones of the created plurality of conductive paths are thereby further in contact with each other at oppositely disposed contact locations. The method of manufacturing a malleable electrode further comprises the step of coating at least parts of the plurality of conducting paths with a coating.

The creating of the plurality of conducting paths further comprises a step of printing the plurality of conducting paths using a silver-containing material.

The coating of at least parts of the plurality of conducting paths further comprises a step of printing using carbon-containing material.

### DESCRIPTION OF THE FIGURES

FIG. 1 shows a view of a first aspect of a malleable electrode.
FIG. 2 shows a cross-sectional view of the first aspect of the malleable electrode.
FIG. 3 shows a view of a first aspect of a clothing comprising the malleable electrode.
FIG. 4 shows a view of a second aspect of the clothing comprising the malleable electrode.
FIG. 5 shows a view of a third aspect of the clothing comprising the malleable electrode.
FIG. 6 shows a cross-sectional view of one of the aspects of the clothing comprising the malleable electrode.
FIG. 7 shows a flow chart describing a method for manufacturing a malleable electrode.

### DETAILED DESCRIPTION OF THE INVENTION

The invention will now be described on the basis of the figures. It will be understood that the embodiments and aspects of the invention described herein are only examples and do not limit the protective scope of the claims in any way. The invention is defined by the claims and their equivalents. It will be understood that features of one aspect or embodiment of the invention can be combined with a feature of a different aspect or aspects and/or embodiments of the invention.

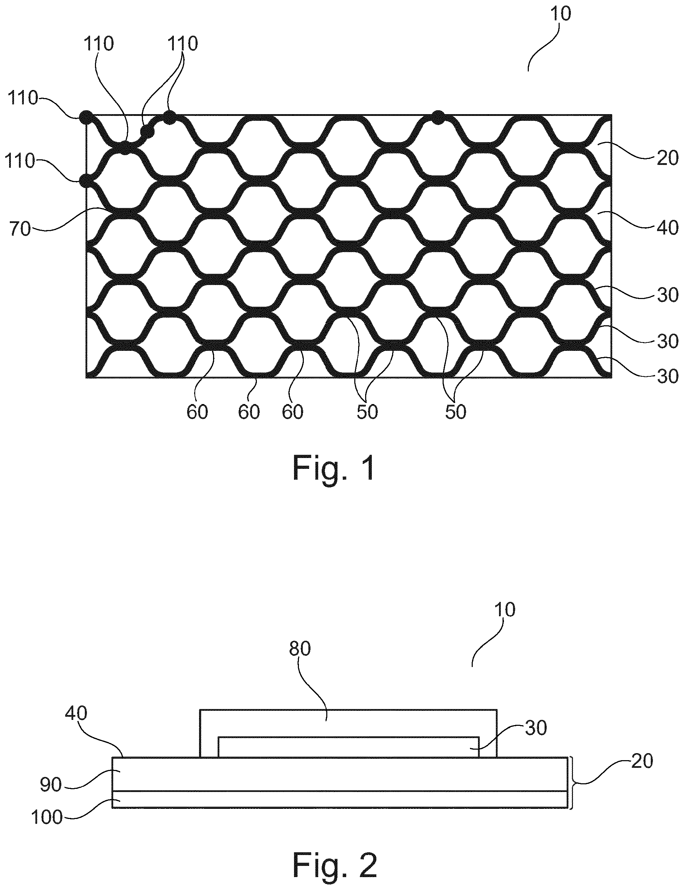

FIG. 1 shows a view of a first aspect of a malleable electrode 10. A malleable substrate 20 forms the base for the malleable electrode 10. The malleable substrate 20 has a plate-shaped form with a spread in two spatial dimensions being significantly larger than in the third spatial dimension. The substrate 20 therefore has two surfaces that are significantly greater in size than the remaining four (side) surfaces of the substrate 20. In this context the term "malleable" is used to indicate a material that can be easily stretched in two dimensions and bent or folded.

The malleable substrate 20 can be perforated with a plurality of holes. The holes can vary in size and number and can be arranged in an even distribution or can be randomly arranged on the malleable substrate 20. The holes allow air to pass through the malleable substrate 20.

The malleable electrode 10 further comprises a plurality of conducting paths 30 that are disposed on a surface 40 of the substrate 20. The surface 40 of the substrate 20 is one of the two bigger surfaces of the substrate 20.

The conductive paths 30 have a wave-shaped form with a plurality of apexes 60 and a wavelength. Adjacent ones of the conductive paths 30 are lengthwise offset to each other by half of the wavelength of their wave-shaped form. The conductive paths 30 are disposed on the surface 40 so that adjacent conductive paths 30 are in contact with each other at contact locations 50. In this case the contact locations 50 lie at opposing apexes 60. The conductive paths 30 thus form a two-dimensional grid-structure 70 with a uniform pattern.

The meander-like wave-shaped form of the conductive paths of Fig. 1 allows the electrode 20 to be malleable without damaging the conductive paths 30. The conductive paths 30 do not tear apart or are damaged if the substrate 20 is subject to loads such as but not limited to stretching, bending or torsion.

The conductive paths 30 can have other forms than the wave-shaped forms shown in Fig. 1. These could be other non-linear forms that allow contact between adjacent conductive paths 30 at multiple contact locations 50, thereby forming a two-dimensional grid-like structure. The two-dimensional grid-like structure does not need to have a uniform pattern.

The conductive paths 30 are made of silver-containing material or other kinds of material with a comparable high electrical conductivity of at least 20 • 10⁶ S/m, such as but not limited to copper or gold. Using a material with high electrical conductivity allows electrical current to flow through the conducting paths 30. The conductive paths 30 can be printed onto a substrate 20 using silver-containing ink. The conductive paths 30 can alternatively be applied to the substrate 20 by any other manufacturing process such as but not limited to stitching or bonding. The substrates 20 have typically a thickness of between 1.5 um and 300 µm, typically between 15 um and 100 µm, but this is not limiting of the invention.

The conductive paths 30 can be manufactured with a thin layer thickness between 1 µm and 50 µm by printing the conductive paths 30 on to the substrate 20. The thin layer thickness of the conductive paths 30 further enhances the malleability of the electrode 10. The thin layer thickness further enhances the material efficiency, as less material is needed for manufacturing the conductive paths 30.

For the malleable electrode 10 to be used as an electrode, the conductive paths 30 need to be electrically connected to electrical devices, such as but not limited to a power source 230 or a measurement device 240. The conductive paths 30 of the malleable electrode 10 can be electrically connected at random connection locations 110. Electrical current is able to flow between any part of the grid-structure 70 and any random connection location 110, as the conductive paths 30 are in contact with each other and form the grid-structure 70. In other words, electrical current can flow between any of the plurality of conductive paths 30 of the malleable electrode 10.

The electrode 10 can be tailored to a desired size. There are no specific connection locations 110 for contacting the electrode and the conductive paths 30 are interconnected at multiple locations, forming the grid-structure 70. The electrode 10 can thus be tailored by e.g., cutting the electrode 10 into pieces of a desired size. It is therefore also possible that the pieces of the electrode 10 have different shapes like e.g., a circular shape or any kind of freeform shape.

FIG. 2 shows a cross-sectional view of the first aspect of the malleable electrode 10. The malleable substrate 20 consists of two layers. The malleable substrate 20 comprises a first layer 90 which is made of a thermoplastic polyurethane. The first layer 90 can also be made of other materials such as but not limited to polyurethane, polyimides, polyethylene naphthalate, polyethylene terephthalate, leather, imitation leather, rubber and silicone. The first layer 90 can also be made of any kind of textile material.

The malleable substrate 20 further comprises a second layer 100, which is made of hotmelt adhesives. The second layer 100 has a thickness of typically between 20 um and 50 um and bonds the malleable electrode 10 to another object, e.g., to an item of clothing 200. The thickness of the second layer 100 can be increased if necessary. The malleable electrode 10 can be applied on to the textile material by heating the hotmelt adhesive of the second layer 100 and bringing the melted hotmelt adhesive into contact with the textile material. The second layer 100 can be made of any other material that allows bonding to other objects. The second layer 100 can be made of other plastic materials such as but not limited to thermoplastics or adhesives. The second layer 100 can also comprise means for establishing a form fit, such as but not limited to hook and loop fasteners. The number of layers of the malleable substrate 20, the materials of which the layers are made, and the arrangement of the layers can be varied if needed or desired.

If the malleable substrate 20 comprises a single layer only, the surface 40 on which the conducting paths 30 can be disposed is on one of the two bigger surfaces of the substrate 20. In case of the shown first aspect of the invention, the substrate 20 includes a first layer 90 and a second layer 100. The conducting paths 30 are disposed on the surface 40 of the first layer 90 of the substrate 20. The surface 40 is opposite of the surface of the first layer 90 that is in contact with the second layer 100 of the substrate 20.

The conducting paths 30 are coated with a coating 80. The coating 80 covers at least part of the surface (and normally all) of the conducting paths 30, so that no direct contact between the conducting paths 30 and anything other than the electrode 10is possible. It is possible that only parts of the conductive paths 30 are covered by the coating 80. The coating 80 is made in one aspect of a carbon-containing material. The coating 80 can alternatively be made of other material with an electric conductivity that is comparable to the electric conductivity of carbon. Carbon can have an electric conductivity between 0,01 and 3 • 10⁶ S/m, which is lower than the very high electric conductivity of metals like silver, copper or gold (more than 20 • 10⁶ S/m).

If the electrode 10 is used as an electrode 10 to be applied on the skin 220 of a human, the resistance between the conductive paths 30 and the skin 220 is high enough due to the used carbon-containing material to avoid an uncomfortably or painfully large electrical current between the electrode 20 and the human body. It is known that a large electrical current passing through the skin can be uncomfortable or even painful. The electrode 10 further provides an electrically conductive path through the grid-structure 70 formed by the silver-containing conductive paths 30 to allow the electric current to flow in the conductive paths 30 without substantial resistive loss. The electrode 10 also provides a suitable resistance between the electrode 10 and e.g., the skin 220 of a human due to the carbon-containing coating 80.

The coating 80 can be printed on the surface of the conducting paths 30 and on the substrate 20 using carbon-containing ink. The coating 80 can alternatively be applied to the conducting paths 30 and to the substrate 20 by any other manufacturing process such as, but not limited to spraying, slot-die coating or applying the coating 80 using a squeegee.

The coating 80 can be manufactured with a thin layer thickness between 1 µm and 50 µm by using the printing process. The coating 80 can also have a layer thickness of up to a maximum of 300 µm if a different manufacturing process is used. The thin layer thickness of the coating 80 further enhances the malleability of the electrode 20. The thin layer thickness further enhances the material efficiency, as less material is needed for manufacturing the coating 80.

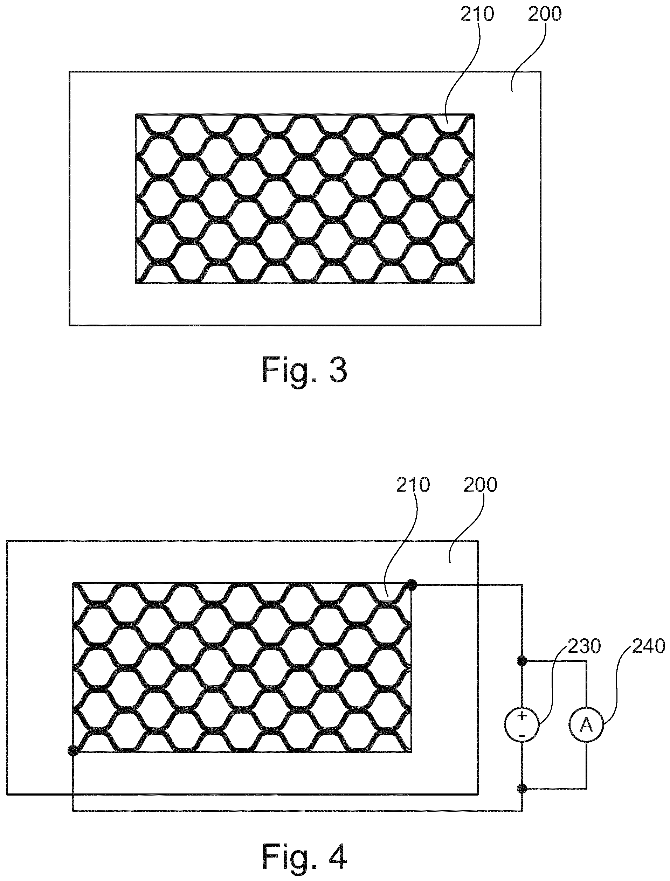

FIG. 3 shows a view of a first aspect of an item of clothing 200 comprising the malleable electrode 10. The same reference signs are used for similar features shown in Fig. 3 as those in Figs. 1 and 2. The item of clothing 200 includes a patch 210 of the textile electrode 10. The shape, size, and number of patches 210 of the electrode 10 can be varied if needed or desired. A typical size for a patch would be 2.5 cm², but this is not limiting of the invention and indeed patches of up to 40cm x 50 cm would be need for the back of an item of clothing.

The patch 210 is arranged on a surface of the clothing 200, so that the electrode 10 contacts the skin 220 of a wearer of the clothing 200 when wearing the clothing 200. The conductive paths 30 and / or the coating 80 come into contact with the skin 220 of the wearer of the clothing 200 when wearing the clothing 200. The patch 210 can be arranged at a location of the clothing 200 so that the electrode 10 contacts the skin 220 adjacent to a muscle.

The patch 210 is bonded to the clothing 200 by the hotmelt adhesive of the second layer 100 of the substrate 20. The patch 210 can alternatively be bonded in any other kind to the textile of the clothing, e.g., by stitching, other adhesive materials or the like.

The substrate 20 of the electrode 10 of the patch 210 can be perforated as described for FIG. 1. This perforation makes wearing a clothing 200 with the malleable electrode 10 more comfortable, as the perforations reduce sweating at the contact area between the skin 220 and the malleable electrode 10 by allowing air to pass through the malleable electrode 10 to the skin

FIG. 4 shows a view of a second aspect of the clothing 200 with the malleable electrode 10. The same reference signs are used for similar features shown in Fig. 4 as those in Figs. 1 to 3.

The clothing 200 further includes a power source 230 with two ports. The power source 230 is electrically connected to the electrode 10. The two ports of the power source 230 are connected to different contacting locations 110 of the electrode 10. For example, the two ports of the power source 230 are connected to two contacting locations 110 that are located on opposite directions of the electrode 10. The electrical current will flow through the grid-structure 70 formed by the conductive paths 30 if a voltage is applied between the different contacting locations 110 through the ports of the power source 230. This configuration allows to use the electrode 10 as a heating element, as the conducting paths 30 will heat due to the electrical resistance.

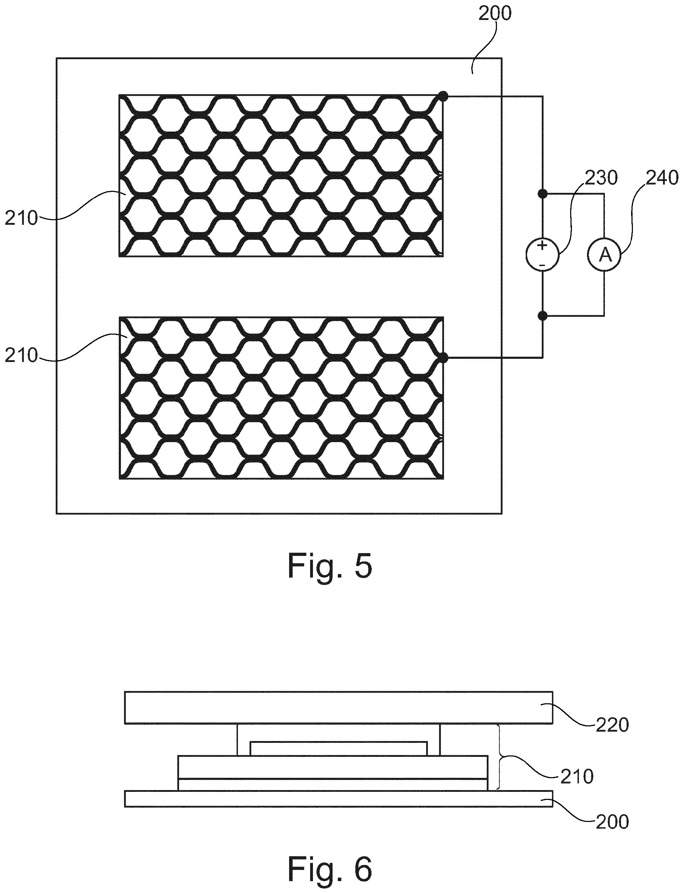

FIG. 5 shows a view of a third aspect of the clothing 200 with the malleable electrode 10. The same reference signs are used for similar features shown in Fig. 5 as those in Figs. 1 to 4.

The clothing includes two patches 210 of the electrode 10. The two patches 210 are arranged on a surface of the clothing, so that the electrodes 10 of the patches 210 contact the skin 220 of a wearer of the clothing 220 when wearing the clothing 220. In one aspect the two patches 210 are located adjacent to the ends of a muscle of the wearer of the clothing 200 when wearing the clothing 200.

The electrode 10 of one patch 210 is electrically connected to one port of the power source 230. The electrode 10 of the other one of the two patches 210 is electrically connected to the other one of the ports of the power source 230. An electrical current can flow from one electrode 10 through the skin 220 to the other electrode 10, if a voltage is applied between the ports of the power source 230. Muscles adjacent to the skin 220 where the two electrodes 10 of the two patches 210 are in contact with the skin 220 can be stimulated by the electrical current.

Alternatively, or additionally a measurement device 240 can be electrically connected to the two electrodes 10. The electrode 10 of one patch 210 is electrically connected to one port of the measurement device 240. The electrode 10 of the other one of the two patches 210 is electrically connected to the other one of the ports of the measurement device 240. This configuration allows the measurement of an electrical current between the two electrodes 10 of the two patches 210, such as, but not limited to measuring the endogenous electrical current with which the contraction of the muscles is triggered.

The shape, size and number of patches 210 of the electrode 10 can be varied if needed or desired. More than two patches 210 can be connected to one power source 230 and / or to one measurement device 240. The clothing 200 can also have more than one power source 230 and / or more than one measurement device 240 that are connected to different patches 210.

FIG. 6 shows a cross-sectional view of one of the aspects of the clothing 200 with the malleable electrode 10. The coating 80 is in contact with the skin of a wearer of the clothing 200 when wearing the clothing.

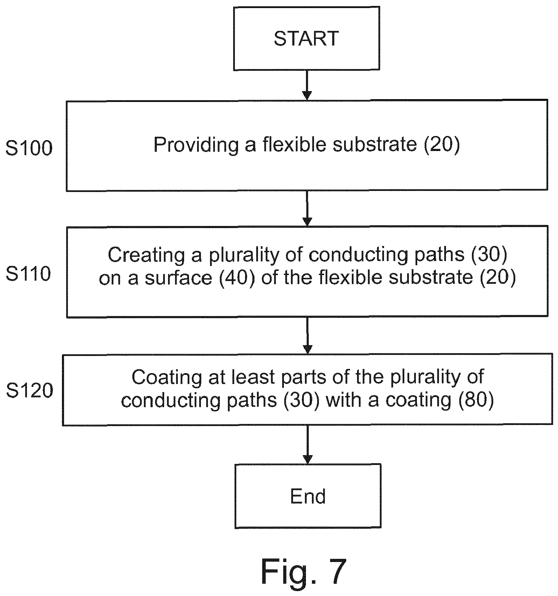

FIG. 7 shows a flow chart describing a method for manufacturing a malleable electrode 10 according to one of the described aspects. A malleable substrate 20 is provided in a step S 100. A plurality of conducting paths 30 are created in a non-linear shape on a surface 40 of the malleable substrate 20 in step S 110. As noted above, adjacent ones of the conducting paths 30 are lengthwise offset from each other and the adjacent conductive paths 30 are in contact with each other at oppositely disposed contact locations 50. Parts or all of the conducting paths 30 are coated with a coating 80 in step S120.

The conducting paths 30 are created on the surface 40 by using a printing manufacturing process in step S110. The material used for printing the conducting paths 30 contains silver (or other materials such as copper or gold) to provide sufficient electrical conductivity.

Parts of the conducting paths 30 are coated with a coating 80 by using a printing manufacturing process in step S 120. As noted above, the material used for printing the coating 80 on to parts of the conducting paths 30 contains carbon but other materials with an electric conductivity that is comparable to the electric conductivity of carbon can be used.

### Reference numerals

- 10: Malleable electrode
- 20: Malleable substrate
- 30: Conducting path
- 40: Surface
- 50: Contact location
- 60: Apex
- 70: Grid structure
- 80: Coating
- 90: First layer
- 100: Second layer
- 110: Connecting location
- 200: Clothing
- 210: Patch
- 220: Skin
- 230: Power Source
- 240: Measurement device
- S100: Providing a malleable substrate
- S110: Creating a plurality of conducting paths on a surface of the malleable sub-strate
- S120: Coating at least parts of the plurality of conducting paths with a coating

## Claims

1. A malleable electrode (10), comprising:
a malleable substrate (20);
a plurality of conducting paths (30), wherein
the plurality of conducting paths (30) are disposed with a non-linear shape on a surface (40) of the substrate (20);
at least two adjacent ones of the plurality of conducting paths (30) are lengthwise offset from each other; and
the at least two adjacent ones of the plurality of conductive paths (30) are in contact with each other at oppositely disposed contact locations (50).

2. The malleable electrode (10) according to claim 1, wherein
the plurality of conducting paths (30) have a wave-shaped form with a plurality of apexes (60); and
adjacent ones of the plurality of conducting paths (30) contact each other at opposing ones of the plurality of apexes (60).

3. The malleable electrode (10) according to claims 1 or 2, wherein
the plurality of conducting paths (30) form a grid structure (70).

4. The malleable electrode (10) according to one of the above claims, wherein
the conducting paths (30) are made of silver-containing material.

5. The malleable electrode (10) according to one of the above claims, wherein
at least ones of the plurality of conducting paths (30) are at least partially coated with a coating (80) made of a carbon-containing material.

6. The malleable electrode (10) according to one of the above claims, wherein
the plurality of conducting paths (30) are printed on the substrate (20) using silver-containing ink.

7. The malleable electrode (10) according to claims 5 or 6, wherein
the coating (80) of the plurality of conducting paths (30) is printed using carbon-containing ink.

8. The malleable electrode (10) according to one of the above claims, wherein
the substrate (20) comprises a first layer (90) made of thermoplastic polyurethane.

9. The malleable electrode (10) according to one of the above claims, wherein
the substrate (20) comprises a second layer (100) made of hotmelt adhesives.

10. The malleable electrode (10) according to one of the above claims, wherein
the substrate (20) is perforated.

11. The malleable electrode (10) according to one of the above claims, wherein
the plurality of conducting paths (30) are connectable at a plurality of locations (110).

12. An item of clothing (200) comprising the malleable electrode (10) according to one of the above claims, wherein at least one patch (210) of the malleable electrode (10) is disposed on an area of the clothing.

13. The item of clothing (200) according to claim 12, wherein
at least one of the plurality of conducting paths (30) is contactable with a skin of a wearer of the clothing (200).

14. The item of clothing (200) according to claim 12, wherein the coating (80) of at least one of the plurality of conducting paths (30) is contactable with a skin of a wearer of the clothing (200).

15. The item of clothing according to one of the claims 12 to 14, further comprising a power source (230) electrically connected to at least one of the plurality of conducting paths (30) of at least one patch (210) of the malleable electrode (10).

16. The item of clothing according to one of the claims 12 to 15, further comprising a current measurement device (240) electrically connected to at least one of the plurality of conducting paths (30) of at least one patch (210) of the malleable electrode (10).

17. A method of manufacturing a malleable electrode, the method comprising the steps of:
providing (S100) a malleable substrate (20);
creating (S110) a plurality of conducting paths (30) on a surface (40) of the malleable substrate (20), such that
the plurality of conducting paths (30) are arranged with a non-linear shape in a plane of the surface (40);
at least two adjacent ones of the plurality of conducting paths (30) are lengthwise offset from each other; and
the at least two adjacent ones of the plurality of conductive paths (30) are in contact with each other at oppositely disposed contact locations (50); and
coating (S 120) at least parts of the plurality of conducting paths (30) with a coating (80).

18. The method according to claim 17, wherein the creating of the plurality of conducting paths (30) comprises a printing using silver-containing material.

19. The method according to one of the claims 17 or 18, wherein the coating of at least parts of the plurality of conducting paths (30) comprises a printing using carbon-containing material.
